# EUROPÄISCHE PATENTANMELDUNG

(11) **EP 4 548 834 A1**
(43) Veröffentlichungstag der Anmeldung: **07.05.2025**
(21) Anmeldenummer: 24208894.6
(22) Anmeldetag: 25.10.2024
(51) Int. Cl.: A61B 5/00

(54) **UNTERSUCHUNGSGERÄT FÜR OPTISCHE MEDIZINISCHE UNTERSUCHUNGEN**

(30) Priorität: 31.10.2023 DE 102023130164
(71) Anmelder: HEINE Optotechnik GmbH & Co. KG, 82205 Gilching (DE)
(72) Erfinder: MICHEL, Felix, 82205 Gilching (DE); HEIN, Holger, 82205 Gilching (DE)
(74) Vertreter: Flach Bauer & Partner Patentanwälte mbB

(57) **Zusammenfassung**

Ein Untersuchungsgerät (10) für optische medizinische Untersuchungen, insbesondere ein Dermatoskop, umfasst eine Kameraeinheit (18) zum Aufnehmen wenigstens eines Bildes eines Untersuchungsbereichs (58) eines Patienten, eine Anzeigevorrichtung (22) und eine Steuerungseinheit (78), wobei die Kameraeinheit (18) ein Sensorelement (72), ein Objektiv (74) und eine Verstelleinrichtung (76) für das Objektiv (74) aufweist, wobei das Objektiv (74) in einem Beobachtungsstrahlengang (70) der Kameraeinheit (18) vor dem Sensorelement (72) angeordnet ist. Die Verstelleinrichtung (76) ist dazu eingerichtet, das Objektiv (74) entlang der optischen Achse des Objektivs (74) in verschiedene Stellpositionen zu verfahren, um verschiedene Untersuchungsebenen im Untersuchungsbereich (58) zu fokussieren, wobei die Steuerungseinheit (78) zur Steuerung der Verstelleinrichtung (76) ausgebildet und mit dieser verbunden ist, und in der Steuerungseinheit (78) Informationen zu zwei oder mehr vordefinierten Untersuchungsebenen (60, 62) im Untersuchungsbereich (58) mit zugehörigen Stellpositionen des Objektivs (74) hinterlegt sind. Die Anzeigevorrichtung (22) ist dazu eingerichtet, eine Darstellung des von der Kameraeinheit (18) aufgenommenen wenigstens einen Bildes anzuzeigen.

Ferner ist ein Verfahren zur Erfassung eines Bildes eines Untersuchungsbereichs angegeben.

## Beschreibung

Die Erfindung betrifft ein Untersuchungsgerät für optische medizinische Untersuchungen, insbesondere ein Dermatoskop.

Optische Untersuchungsgeräte, insbesondere Dermatoskope, sind bekannt und weisen üblicherweise eine Optik sowie eine Beleuchtungseinrichtung auf, mit der ein Untersuchungsbereich beleuchtet werden kann. Zudem sind optische Untersuchungsgeräte erhältlich, die über eine integrierte Kamera verfügen, die dazu eingerichtet ist, Bilder des Untersuchungsbereichs aufzunehmen und auf einer Anzeige des optischen Untersuchungsgeräts darzustellen.

Verschiedene Körperstellen eines zu untersuchenden Patienten unterscheiden sich hinsichtlich der Art und Ausdehnung eines gewünschten Untersuchungsbereichs. Beispielsweise weisen Teilschichten der menschlichen Haut, wie die Epidermis (Oberhaut) und die Dermis (Lederhaut) unterschiedliche Dicken auf, je nachdem, ob sich das betrachtete Teilstück der Haut auf der Brust, dem Kopf oder dem Handrücken des jeweiligen Patienten befindet. Soll eine bestimmte Hautschicht mittels des optischen Untersuchungsgeräts betrachtet werden, muss daher anpassbar sein, welche Teilschicht der Haut mittels der Optik fokussiert wird und somit in einer möglichst scharfen Darstellung betrachten zu können.

Zu diesem Zweck ist es bekannt, dass eine Bedienperson des Untersuchungsgeräts, insbesondere ein Arzt, manuell den Fokus der Kamera einstellen kann, um die gewünschte Hautschicht im zu betrachtenden Untersuchungsbereichs scharf zu stellen.

Dieser Vorgang ist jedoch mit erheblichem Aufwand für die Bedienperson des Untersuchungsgeräts verbunden und verlängert Untersuchungszeiten, wodurch weniger Zeit für ein Patientengespräch zur Verfügung steht.

Es ist daher Aufgabe der Erfindung, ein Untersuchungsgerät für optische medizinische Untersuchungen bereitzustellen, das einfach in der Bedienung ist und es insbesondere ermöglicht, auf einfache und reproduzierbare Weise Aufnahmen eines Untersuchungsbereichs in hoher Qualität zu erhalten.

Die Aufgabe wird gelöst durch ein Untersuchungsgerät für optische medizinische Untersuchungen, insbesondere ein Dermatoskop, mit einer Kameraeinheit zum Aufnehmen wenigstens eines Bildes eines Untersuchungsbereichs eines Patienten, einer Anzeigevorrichtung und einer Steuerungseinheit, wobei die Kameraeinheit ein Sensorelement, ein Objektiv und eine Verstelleinrichtung für das Objektiv aufweist, und wobei das Objektiv in einem Beobachtungsstrahlengang der Kameraeinheit vor dem Sensorelement angeordnet ist. Die Verstelleinrichtung ist dazu eingerichtet, das Objektiv entlang der optischen Achse des Objektivs in verschiedene Stellpositionen zu verfahren, um verschiedene Untersuchungsebenen im Untersuchungsbereich zu fokussieren. Die Steuerungseinheit ist zur Steuerung der Verstelleinrichtung ausgebildet und mit dieser verbunden, und in der Steuerungseinheit sind Informationen zu zwei oder mehr vordefinierten Untersuchungsebenen im Untersuchungsbereich mit zugehörigen Stellpositionen des Objektivs hinterlegt. Die Anzeigevorrichtung ist dazu eingerichtet, eine Darstellung des von der Kameraeinheit aufgenommenen wenigstens einen Bildes anzuzeigen.

Die Erfindung basiert auf dem Gedanken, ein Untersuchungsgerät bereitzustellen, das über vorgegebene bzw. vordefinierte Stellpositionen eine wenigstens teilautomatisierte, bevorzugt vollautomatisierte Anpassung der Position des Objektivs im Beobachtungsstrahlengang ermöglicht, sodass auch ohne manuelle Justierung des Untersuchungsgeräts verschiedene Untersuchungsebenen untersucht werden können. Zudem ist sichergestellt, dass eine gewünschte Untersuchungsebene im Untersuchungsbereich scharf dargestellt wird.

Dadurch, dass in der Steuerungseinheit die vordefinierten Untersuchungsebenen mit zugehörigen Stellposition verknüpft sind und zugleich die Steuerungseinheit dazu eingerichtet ist, die Verstelleinrichtung zu kontrollieren, ist sichergestellt, dass durch die Auswahl einer Untersuchungsebene durch die Bedienperson des Untersuchungsgeräts das Objektiv in die korrekte Stellposition verfahren wird, in der die gewünschte Untersuchungsebene scharf gestellt wird.

Der Begriff "Untersuchungsbereich" bezeichnet hier und im Folgenden beispielsweise einen dreidimensionalen Abschnitt des zu untersuchenden Patienten, der als Abfolge von übereinander angeordneten Untersuchungsebenen bzw. Schnitten aufgefasst werden kann.

Die zur jeweiligen Untersuchungsebene zugehörige Stellposition des Objektivs ist insbesondere derart bestimmt, dass das Objektiv die Untersuchungsebene auf das Sensorelement scharf abbildet, wenn sich das Objektiv in der zur Untersuchungsebene zugehörigen Stellposition befindet.

Die Information zur Untersuchungsebene, die in der Steuerungseinheit hinterlegt ist, ist insbesondere die Tiefe der jeweiligen Untersuchungsebene in der Haut des Patienten, zum Beispiel definiert als Abstand der jeweiligen Untersuchungsebene von der Hautoberfläche des Patienten, wobei die Hautoberfläche über die *Stratum disjunctum* definiert ist, also über den obersten Teil der Homschicht der Epidermis.

Das Untersuchungsgerät ist beispielsweise ein Handgerät.

Beispielsweise ist der Untersuchungsbereich ein Hautbereich des Patienten. Dementsprechend kann das Untersuchungsgerät ein Dermatoskop zum Untersuchen des Hautbereichs des Patienten sein. In derartigen Anwendungen ist es von besonderem Vorteil, dass das Untersuchungsgerät wenigstens teilautomatisiert scharfstellbar ist, um in Abhängigkeit des jeweils untersuchten Hautbereichs des Patienten auf einfache Weise sicherzustellen, dass die Bedienperson des Untersuchungsgeräts ein scharfes Bild der jeweiligen Untersuchungsebene erhält.

Entsprechend können die Informationen zu den Untersuchungsebenen Angaben zum Hauttyp, zum Körperteil, zur Art des Hautbereichs, zur Art der vermuteten Läsion und/oder zur Position eines zu untersuchenden Hautbereichs umfassen.

Die Art des Hautbereichs umfasst beispielsweise die Ethnie, ob es sich um männliche oder weibliche Haut, erkrankte oder gesunde Haut und/oder eine Schleimhaut oder keine Schleimhaut handelt.

Das Körperteil kann beispielsweise das Gesicht, der Oberkörper, der Unterleib, der Rücken, die Hand oder der Fuß des Patienten sein.

Die Position des zu untersuchenden Hautbereichs ist insbesondere über den Ort am jeweiligen Körperteil definiert. Beispielsweise bezeichnet die Position, dass der Hautbereich Teil des Handrückens oder des Fußrückens ist.

In einer Variante umfasst das Untersuchungsgerät eine Benutzerschnittstelle, mit der der Hauttyp, das zu untersuchende Körperteil, die Art des Hautbereichs, die Art der vermuteten Läsion und/oder die Position des zu untersuchenden Hautbereichs auswählbar ist, und wobei die vordefinierten Untersuchungsebenen in Abhängigkeit des ausgewählten wenigstens einen Hauttyps, des zu untersuchenden Körperteils, der Art des Hautbereichs, der Art der vermuteten Läsion und/oder der Position des zu untersuchenden Hautbereichs ausgewählt sind. Auf diese Weise muss der Benutzer des Untersuchungsgeräts lediglich den geplanten Anwendungsort über zumindest eine der zuvor definierten Parameter über die Benutzerschnittstelle angeben, um die zugehörige Untersuchungsebene scharf zu stellen, und muss keine manuelle Justierung vornehmen.

Insbesondere ist auf diese Weise eine intuitive Benutzung des Untersuchungsgeräts ermöglicht, da vom Benutzer keine Angabe von abstrakten bzw. numerischen Werten zur Lage bzw. Tiefe der gewünschten Untersuchungsebene im Untersuchungsbereich über die Benutzerschnittstelle eingeben muss, sondern auf einfach zu erfassende Begriffe zur Auswahl der gewünschten Untersuchungsebene zurückgreifen kann.

Um die Bedienung des Untersuchungsgeräts noch weiter zu erleichtern, kann die Anzahl verfügbarer Optionen über die Benutzerschnittstelle eingeschränkt sein. Beispielsweise wird lediglich eine begrenzte Anzahl an Standardeinstellungen über die Benutzerschnittstelle angeboten, die voreingestellten Messszenarien entsprechen.

Beispielhaft können die Standardeinstellungen die Messszenarien "Gesicht", "Oberkörper", "Handrücken" und "Schleimhaut" vorgeben, jeweils in einer Variante für männliche Patienten und für weibliche Patienten.

Bevorzugt ist das Untersuchungsgerät dazu eingerichtet, den Hauttyp, das zu untersuchende Körperteil, die Art des Hautbereichs, die Art der vermuteten Läsion und/oder die Position des zu untersuchenden Hautbereich zu erkennen und/oder auszuwählen. Auf diese Weise wird die Benutzung des Untersuchungsgeräts noch weiter vereinfacht, da die Bedienperson des Untersuchungsgeräts nicht mehr selbst den Untersuchungsbereich über die Benutzerschnittstelle vornehmen muss. Anders ausgedrückt stellt sich das Untersuchungsgerät bevorzugt wenigstens teilweise automatisiert auf eine zu untersuchende Untersuchungsebene scharf, insbesondere vollautomatisiert.

Insbesondere ist das Untersuchungsgerät dazu eingerichtet den Hauttyp, das zu untersuchende Körperteil, die Art des Hautbereichs, die Art der vermuteten Läsion und/oder die Position des zu untersuchenden Hautbereichs anhand eines aufgenommenen Bildes der Kameraeinheit oder eines aufgenommenen Bildes einer zweiten Kameraeinheit des Untersuchungsgeräts zu erkennen und/oder auszuwählen.

Beispielsweise erkennt das Untersuchungsgerät, insbesondere die Steuereinheit, den Hauttyp, das zu untersuchende Körperteil, die Art des Hautbereichs, die Art der vermuteten Läsion und/oder die Position des zu untersuchenden Hautbereichs anhand eines aufgenommenen Bildes, das mittels der Kameraeinheit aufgenommen wurde, die auf die äußere Oberfläche der Kontaktscheibe bzw. der Hautoberfläche fokussiert war.

Die zweite Kameraeinheit weist in diesem Zusammenhang insbesondere eine von der ersten Kameraeinheit abweichende Blickrichtung auf.

Der wenigstens eine Hauttyp kann durch das Körperteil, die Art des Hautbereichs, die Art der vermuteten Läsion und/oder die Position des Hautbereichs am Patienten vorgegeben sein. Anders ausgedrückt kann der Hauttyp durch wenigstens eine weitere Information zur Untersuchungsebene festgelegt sein.

Die Verstelleinrichtung kann ein Aktuator sein, insbesondere ein Schrittmotor oder ein Voice-Coil-Motor. Derartige Verstelleinrichtungen erlauben eine besonders präzise Kontrolle über die Position des Objektivs entlang der optischen Achse, weisen lediglich einen geringen Bauraumbedarf auf und sind kostengünstig verfügbar.

In einer weiteren Variante umfasst das Untersuchungsgerät eine Benutzerschnittstelle, mit der eine der vordefinierten Untersuchungsebenen auswählbar ist, und wobei die Steuerungseinrichtung dazu ausgebildet ist, das Objektiv in die zur gewählten Untersuchungsebene zugehörige Stellposition zu verfahren, insbesondere wobei lediglich solche vordefinierten Untersuchungsebenen zur Auswahl über die Benutzerschnittstelle angeboten werden und/oder auswählbar sind, die dem bzw. der zuvor ausgewählten Hauttyp, Körperteil, Art der vermuteten Läsion, Art und/oder Position eines zu untersuchenden Hautbereichs zugeordnet sind. Auf diese Weise wird eine besonders einfache Bedienung des Untersuchungsgeräts ermöglicht, indem der Bedienperson angeboten wird, die Untersuchungsebene direkt auszuwählen, wobei insbesondere lediglich diejenige Unterauswahl der Untersuchungsebenen angezeigt wird, die einer zuvor bereits getätigten Einschränkung durch die Bedienperson bzw. durch das Untersuchungsgerät selbst entspricht.

Zur Interaktion mit der Bedienperson des Untersuchungsgeräts kann die Benutzerschnittstelle wenigstens eine Taste und/oder ein berührungsempfindliches Display umfassen.

Bei der Benutzerschnittstelle zum Auswählen einer der vordefinierten Untersuchungsebenen handelt es sich insbesondere um die gleiche Benutzerschnittstelle, mit der der Hauttyp, das zu untersuchende Körperteil, die Art des Hautbereichs, die Art der vermuteten Läsion und/oder die Position des zu untersuchenden Hautbereichs auswählbar sind.

Die Anzeigevorrichtung kann dazu eingerichtet sein, die Darstellung des von der Kameraeinheit aufgenommenen wenigstens eines Bildes als Live-Darstellung anzuzeigen. Entsprechend kann es sich bei dem Untersuchungsgerät insbesondere um ein digitales Dermatoskop handeln.

Weist die Benutzerschnittstelle ein berührungsempfindliches Display auf, kann das berührungsempfindliches Display Teil der Anzeigevorrichtung sein. Auf diese Weise resultiert eine besonders kompakte Bauform des Untersuchungsgeräts.

In einer weiteren Variante umfasst das Untersuchungsgerät eine Benutzerschnittstelle, mittels der die Stellposition des Objektivs derart einstellbar ist, dass die Stellposition des Objektivs um einen vorgegebenen Wert oder ein Vielfaches des vorgegebenen Werts von der aktuellen Stellposition verändert wird. Auf diese Weise können zusätzliche Untersuchungsebenen um die vordefinierte Untersuchungsebene herum fokussiert werden, wobei die jeweiligen zusätzlichen Untersuchungsebenen in festen Abständen um die vordefinierte Untersuchungsebene angeordnet sind. Dies ermöglicht es der Bedienperson des Untersuchungsgeräts auf einfache Weise einen dreidimensionalen Eindruck der näheren Umgebung des Untersuchungsbereichs um die vordefinierte Untersuchungsebene herum zu erlangen.

Der durch den vorgegebenen Wert oder das Vielfache des vorgegebenen Werts definierte Abstand der zusätzlichen Untersuchungsebenen ist insbesondere geringer als der Abstand zwischen den durch die verschiedenen Stellpositionen vordefinierten Untersuchungsebenen. Anders ausgedrückt ist über die vordefinierten Stellpositionen eine übergeordnete Auswahl der vordefinierten Untersuchungsebenen möglich, während die Anpassung über die Benutzerschnittstelle eine Feinjustierung um diese vordefinierten Untersuchungsebenen ermöglicht.

In noch einer weiteren Variante ist über die Benutzerschnittstelle die Stellposition des Objektivs manuell stufenlos justierbar. Anders ausgedrückt ist in dieser Ausgestaltung die Stellposition des Objektivs auch ohne von der Bedienperson im Vorfeld festgelegte Untersuchungsebenen bzw. Informationen zur Untersuchungsebene anpassbar. Dies erlaubt es der Bedienperson, insbesondere einer im Umgang mit dem Untersuchungsgerät erfahrenen Bedienperson, eine bedarfsgerechte und zielgenaue Steuerung der fokussierten bzw. scharfgestellten Untersuchungsebene vorzunehmen, ohne von etwaigen in der Steuerungseinheit hinterlegten Informationen und Werten eingeschränkt zu sein und ohne, dass die besonders einfache Grundfunktionalität des Untersuchungsgeräts beeinträchtigt wird.

Die Benutzerschnittstelle zum Verändern der Stellposition des Objektivs ist insbesondere die gleiche Benutzerschnittstelle, die bereits zuvor zum Auswählen der vordefinierten Untersuchungsebene sowie des Hauttyps, des zu untersuchenden Körperteils, der Art des Hautbereichs, der Art der vermuteten Läsion und/oder der Position des zu untersuchenden Hautbereich beschrieben wurde.

Um die Qualität der mittels des Untersuchungsgeräts erhaltenen Bilder weiter zu erhöhen, kann die Steuerungseinheit dazu eingerichtet sein, in einem Kalibrierungsmodus, in dem das Untersuchungsgerät auf eine Referenzfläche aufgesetzt ist, die zwei oder mehr Stellpositionen anhand einer Autofokussierung zu ermitteln, und/oder einen Weißabgleich anhand wenigstens eines mittels der Kameraeinheit aufgenommenen Bildes der Referenzfläche durchzuführen.

In diesem Zusammenhang kann die Stellposition für die Kalibrierung von der den Untersuchungsebenen zugehörigen Stellposition abweichen, wobei die den Untersuchungsebenen zugehörigen Stellposition über eine Umrechnung der bekannten Differenzwerte der Untersuchungsebenen zu einer Kalibrierungsebene, in der sich die Referenzfläche befindet, in Verstellwege des Objektivs ausgehend von der Stellposition für die Kalibrierung bestimmt sind.

Die Referenzfläche enthält insbesondere ein Target, das für die Autofokussierung und/oder den Weißabgleich herangezogen wird. Über den Autofokus wird die Lage der Referenzfläche bzw. Kalibrierungsebene, die in diesem Zusammenhang als Hautoberfläche angesehen wird, bestimmt. Die Autofokussierung wird insbesondere über eine Bestimmung des maximalen Kontrasts im von der Kameraeinheit aufgenommenen Bild in Abhängigkeit der Stellposition des Objektiv ermittelt.

Denkbar ist auch, dass Autofokussierung in an sich bekannter Weise anhand der Phasenverschiebung auf den Zellen des Sensorelementes erfolgt (sog. PDAF - Phase Detection Auto Focus).

Indem im Kalibrierungsmodus ein Weißabgleich durchgeführt werden kann, wird eine besonders zuverlässige Farbgebung der im Einsatz des Untersuchungsgeräts erhaltenen Bilder ermöglicht. Beispielsweise wird die Alterung des Geräts durch den Weißabgleich ausgeglichen. Dies ist insbesondere bei der Untersuchung von Hautbereichen eines Patienten von besonderer Bedeutung.

Die Referenzfläche ist beispielsweise die Oberfläche einer Kalibrierungskarte.

Die Aufgabe wird ferner gelöst durch ein Verfahren zur Erfassung eines Bildes eines Untersuchungsbereichs mittels eines Untersuchungsgeräts, insbesondere eines Untersuchungsgeräts wie zuvor beschrieben, umfassend: Es wird eine Untersuchungsebene ausgewählt und ein Objektiv einer Kameraeinheit des Untersuchungsgeräts auf eine der ausgewählten Untersuchungsebene zugeordneten Stellposition verstellt. Anschließend wird mittels eines Sensorelementes der Kameraeinheit wenigstens ein Bild aufgenommen.

Das aufgenommene Bild kann gespeichert werden und/oder als Live-Darstellung angezeigt werden. Insbesondere bei einer Live-Darstellung ist ein dauerhaftes Speichern nicht notwendig.

Das Auswählen der Untersuchungsebene erfolgt insbesondere durch die Bedienperson oder durch das Untersuchungsgerät. Anders ausgedrückt kann die Untersuchungsebene entweder basierend auf einer Vorgabe bzw. Eingabe der Bedienperson ausgewählt werden oder wenigstens teilweise automatisiert vom Untersuchungsgeräts selbst.

In diesem Zusammenhang wird auf die obigen Ausführungen zum Untersuchungsgerät verwiesen, dessen Merkmale und Eigenschaften auch für das erfindungsgemäße Verfahren gelten und umgekehrt.

Weitere Merkmale und Vorteile der Erfindung ergeben sich aus der nachfolgenden Beschreibung sowie aus den beigefügten Zeichnungen, auf die Bezug genommen wird. In den Zeichnungen zeigen:
- Fig. 1: ein Untersuchungsgerät gemäß einer Ausführungsform der Erfindung in einer perspektivischen Ansicht,
- Fig. 2: eine weitere perspektivische Ansicht des Untersuchungsgeräts gemäß Fig. 1,
- Fig. 3: eine Schnittansicht ausgewählter Teile des Untersuchungsgeräts gemäß Fig. 1,
- Fig. 4: eine erste Darstellung des Untersuchungsgeräts in Fig. 1 in einem erfindungsgemäßen Verfahren zur Erfassung eines Bildes eines Untersuchungsbereichs,
- Fig. 5: eine Darstellung analog zu Fig. 4, wobei ein anderer Untersuchungsbereich betrachtet wird,
- Fig. 6: eine zweite Darstellung des Untersuchungsgeräts analog zu Fig. 4,
- Fig. 7: eine schematische Darstellung einer Referenzfläche zum Kalibrieren des Untersuchungsgeräts aus Fig. 1, und
- Fig. 8: ein Blockschema des erfindungsgemäßen Verfahrens.

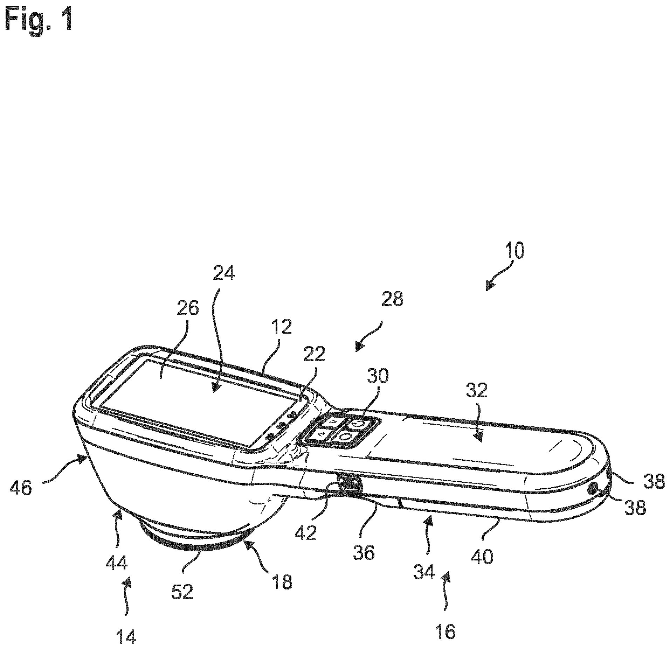

In Figur 1 ist ein Untersuchungsgerät 10 für optische medizinische Untersuchungen dargestellt.

Das Untersuchungsgerät 10 ist beispielsweise ein Dermatoskop und ist in der gezeigten Ausführungsform als Handgerät ausgeführt.

Das Untersuchungsgerät 10 weist ein Gehäuse 12 auf und verfügt über einen Kopfbereich 14 und einen Griffbereich 16, der an den Kopfbereich 14 angrenzt.

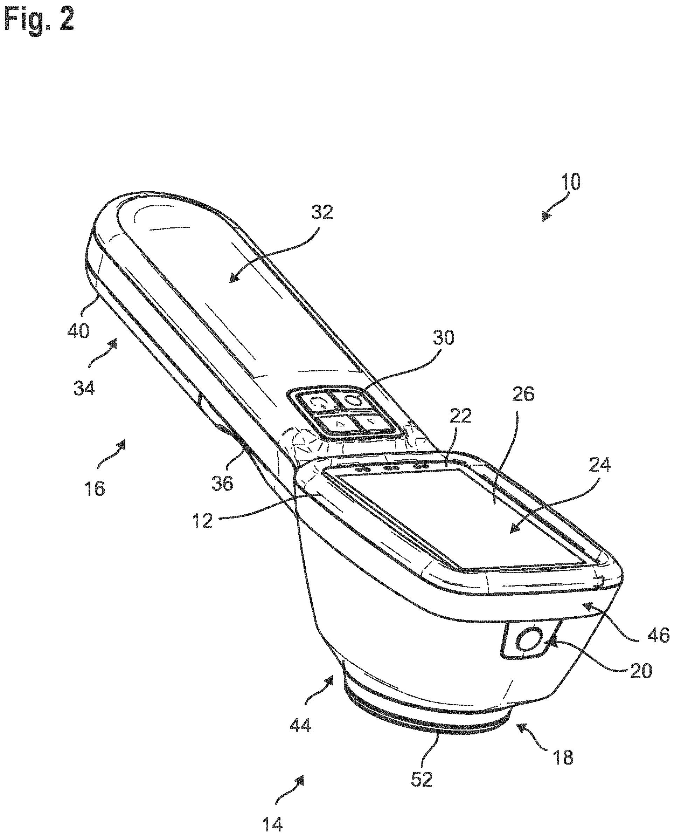

Im Kopfbereich 14 ist eine Kameraeinheit 18 sowie eine zweite Kameraeinheit 20 (vgl. Figur 2) angeordnet, auf die später noch näher eingegangen wird. Die zweite Kameraeinheit 20 ist jedoch optional.

Das Untersuchungsgerät 10 verfügt weiter über eine Anzeigevorrichtung 22, die eine an einer Oberseite 24 des Kopfbereichs 14 angeordnete Anzeige 26 umfasst. Die Anzeige 26 ist dazu eingerichtet, von der Kameraeinheit 18 aufgenommene Bilder anzuzeigen, insbesondere in Form einer Livedarstellung.

Der Griffbereich 16 weist eine Benutzerschnittstelle 28 mit Bedienelementen 30 auf, die in Form von Tasten an einer Oberseite 32 des Griffbereichs 16 angeordnet sind.

Es ist denkbar, dass die Bedienelemente 30 auch anders ausgestaltet sind, beispielsweise als berührungsempfindliches Display. Auch kann die Anzeigevorrichtung 22, nämlich die Anzeige 26, als berührungsempfindliches Display ausgestaltet sein, das zugleich Teil der Benutzerschnittstelle 28 ist. In diesem Fall ist es auch denkbar, dass auf die Bedienelemente 30 in Form von Tasten verzichtet wird.

Entlang des Griffbereichs 16 sind die Bedienelemente 30 an dem dem Kopfbereich 14 zugewandten Ende des Griffbereichs 16 angeordnet, sodass diese von einer Bedienperson des Untersuchungsgeräts 10 beispielsweise mit dem Daumen bedient werden können, wenn diese den Griffbereich 16 mit der Hand ergreift.

An einer Unterseite 34 des Griffbereichs 16 ist zudem ein Verjüngungsabschnitt 36 vorhanden, der derart ausgeformt ist, dass die Bedienperson beispielsweise den Zeigefinger in den Verjüngungsabschnitt 36 einlegen kann, wenn der Griffbereich 16 mit der Hand ergriffen wird. Auf diese Weise ergibt sich eine besonders ergonomische Handhaltung bei der Benutzung des Untersuchungsgeräts 10.

In Figur 1 ist ferner an dem vom Kopfbereich 14 abgewandten Ende des Griffbereichs 16 eine Ladschnittstelle 38 vorhanden. Die Ladeschnittstelle 38 dient dazu, einen innerhalb des Gehäuses 12 integrierten (nicht dargestellten) Energiespeicher elektrisch aufzuladen.

Beispielsweise ist das Untersuchungsgerät 10 Teil einer Untersuchungsgerätebaugruppe, die das Untersuchungsgerät 10 und eine Ladestation für das Untersuchungsgerät 10 umfasst. Die Ladestation kann in diesem Fall eine Aufnahme aufweisen, in die das Untersuchungsgerät 10 mit dem die Ladeschnittstellen 38 aufweisenden Ende des Griffbereichs 16 zerstörungsfrei lösbar eingesteckt wird, um einen Ladevorgang durchzuführen.

Grundlegend ist es auch möglich, dass anstelle eines wiederaufladbaren Energiespeichers, also eines Akkumulators, eine einmalig nutzbare Batterie als austauschbarer Energiespeicher vorhanden ist.

Um einen Austausch des integrierten Energiespeichers zu ermöglichen, verfügt der Griffbereich über ein zerstörungsfrei abnehmbares Abdeckelement 40, das beispielsweise am Gehäuse 12 über eine Rastverbindung befestigbar ist. Indem das Abdeckelement 40 entfernt wird, kann der austauschbare Energiespeicher entnommen und ausgetauscht werden.

In Figur 1 ist zudem zu erkennen, dass im Griffbereich 16 eine Kommunikationsschnittstelle 42 vorgesehen ist, die in der gezeigten Ausführungsform als USB-C-Schnittstelle ausgeführt ist. Über die Kommunikationsschnittstelle 42 kann das Untersuchungsgerät 10 mit einem externen computergestützten Datenauswertungssystem verbunden werden, insbesondere, um von der Kameraeinheit 18 und/oder der zweiten Kameraeinheit 20 aufgenommene Bilder zu übertragen.

Es ist denkbar, dass die Kommunikationsschnittstelle 42 auch anders ausgeführt ist, beispielsweise als Schnittstelle zur drahtlosen Kommunikation.

Ferner kann die Kommunikationsschnittstellte 42 mit dem integrierten Energiespeicher elektrisch leitend verbunden sein, sodass der integrierte Energiespeicher auch über die Kommunikationsschnittstelle 42 elektrisch aufladbar ist.

In Figur 1 ist weiter zu erkennen, dass der Griffbereich 16 unter einem Winkel an den Kopfbereich 14 angeformt ist. Die Ebenen, die durch die Oberseite 24 des Kopfbereichs 14 und die Oberseite 32 des Griffbereichs 16 definiert sind, stehen in einem Winkel zueinander. Auf diese Weise ist die Ergonomie des Untersuchungsgeräts 10 weiter verbessert.

Figur 2 zeigt eine weitere perspektivische Darstellung des Untersuchungsgeräts 10, in der die Anordnung der Kameraeinheit 18 und der zweiten Kameraeinheit 20 besser zu erkennen ist.

Die Kameraeinheit 18 ist einer Unterseite 44 des Kopfbereichs 14 zugeordnet, nämlich in die Unterseite 44 integriert. Die Integration ist derart, dass die Blickrichtung der Kameraeinheit 18 nach unten verläuft, in der gezeigten Ausführungsform also im Wesentlichen senkrecht zur Oberseite 24 des Kopfbereichs 14.

Im Gegensatz dazu ist die zweite Kameraeinheit 20 einer Vorderseite 46 des Kopfbereichs 14 zugeordnet, nämlich in die Vorderseite 46 integriert. Die Integration ist derart, dass die Blickrichtung der zweiten Kameraeinheit 20 nach vorne verläuft, also im Wesentlichen parallel bzw. geringfügig geneigt zur Oberseite 24 des Kopfbereichs 14.

Grundlegend kann das Untersuchungsgerät 10 auch lediglich die Kameraeinheit 18 aufweisen, also keine zweite Kameraeinheit 20 umfassen.

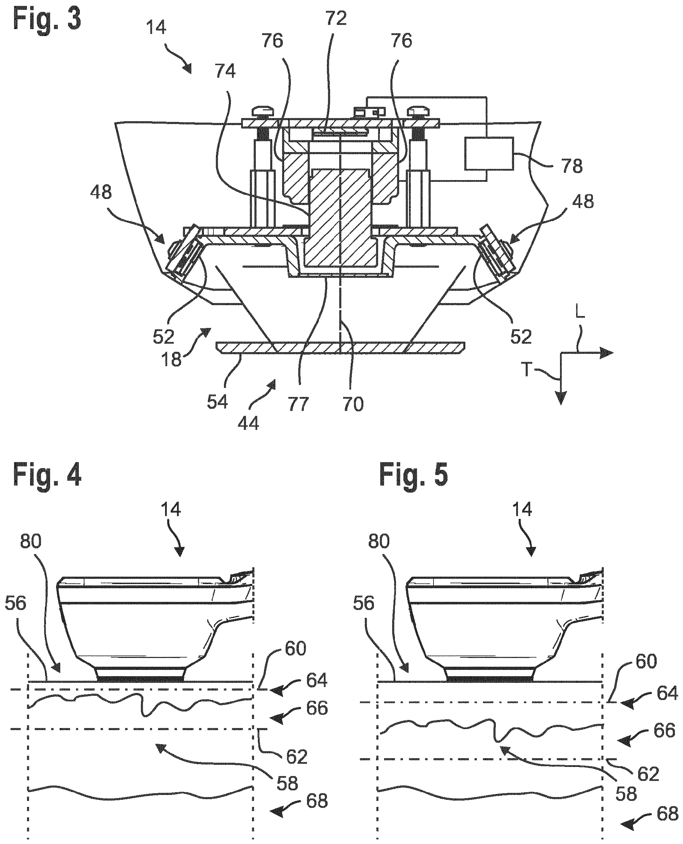

Figur 3 zeigt eine Schnittansicht ausgewählter Teile des Untersuchungsgeräts 10, speziell des Kopfbereichs 14, um den Aufbau und die Funktionsweise der Kameraeinheit 18 besser zu verdeutlichen. Die Schnittebene in Figur 3 verläuft parallel zu einer Ebene, die durch die Längsrichtung L des Untersuchungsgeräts 10 und eine Tiefenrichtung T definiert ist, die senkrecht zur Längsrichtung L verläuft.

Die Kameraeinheit 18 umfasst mehrere Beleuchtungseinrichtungen 48, die beispielsweise als lichtemittierende Dioden (LEDs) ausgestaltet sind und dazu dienen, Licht in Richtung einer Kontaktscheibe 54 zu emittieren.

Das von den Beleuchtungsvorrichtungen 48 emittierte Licht ist Licht einer vorgegebenen Wellenlänge oder eines vorgegebenen Wellenlängenspektrums.

Zwischen einzelnen der Beleuchtungsvorrichtungen 48 und der Kontaktscheibe 54 sind zudem Polarisationsfilter 52 vorgesehen.

Die Beleuchtungsvorrichtungen 48 mit vorgesehenem Polarisationsfilter 52 können unabhängig von den Beleuchtungsvorrichtungen 52 ohne Polarisationsfilter 52 geschaltet werden, sodass der Untersuchungsbereich wahlweise mit polarisiertem Licht oder unpolarisiertem Licht ausgeleuchtet werden kann.

Die Kameraeinheit 18 schließt an der Unterseite 44 des Kopfbereichs 14 mit der Kontaktscheibe 54 ab, die bei Einsatz des Untersuchungsgeräts 10 auf die Hautoberfläche 56 eines zu untersuchenden Patienten aufgelegt wird (vgl. Figur 4).

Durch die Positionierung der Kontaktscheibe 54 auf dem Patienten wird ein Untersuchungsbereich 58 des Patienten definiert, der sich, ausgehend von der Hautoberfläche 56, aus einer Vielzahl von entlang der Tiefenrichtung T aufeinanderfolgenden Untersuchungsebenen zusammensetzt, von denen in Figur 4 zur Veranschaulichung lediglich zwei vordefinierte Untersuchungsebenen 60 und 62 mit Strichpunktlinien angedeutet sind.

Der Untersuchungsbereich 58 erstreckt sich dabei von der Hautoberfläche 56, die über die *Stratum disjunctum* definiert ist, und die an der Kontaktscheibe 54 anliegt, über die Epidermis 64 (auch als "Oberhaut" bezeichnet) zur Dermis 66 (auch als "Lederhaut" bezeichnet). Unterhalb der Dermis 66 ist die Subkutis 68 angeordnet (auch als "Unterhaut" bezeichnet), die üblicherweise nicht betrachtet werden muss, jedoch optional ebenfalls Bestandteil des Untersuchungsbereichs 58 sein kann.

Das von den Beleuchtungseinrichtungen 48 emittierte Licht dringt über die Kontaktscheibe 54 in die Haut des Patienten, speziell in den Untersuchungsbereich 58, ein und wird von dieser bzw. diesem zumindest teilweise in Richtung der Kameraeinheit 18 entlang eines Beobachtungsstrahlengangs 70 reflektiert (vgl. Figur 3).

Der Beobachtungsstrahlengang 70 umfasst beispielsweise alles Licht, das zum Sensorelement 72 gelangt.

Um dieses reflektierte Licht zu detektieren, verfügt die Kameraeinheit 18 über ein Sensorelement 72 sowie ein Objektiv 74, wobei das Objektiv 74 entlang des Beobachtungsstrahlengangs 70 vor dem Sensorelement 72 angeordnet ist und dazu dient, einfallendes Licht auf das Sensorelement 72 zu fokussieren.

Das Sensorelement 72 ist beispielsweise ein CCD-Sensor oder CMOS-Sensor.

Das Objektiv 74 ist ferner mit einer Verstelleinrichtung 76 verbunden, die dazu eingerichtet ist, dass Objektiv 74 entlang der optischen Achse des Objektivs 74 in verschiedene Stellpositionen zu verfahren, das heißt in Axialrichtung des Objektivs 74.

Die Verstelleinrichtung 76 ist im gezeigten Ausführungsbeispiel ein Voice-Coil-Motor. Grundlegend können jedoch auch andere Arten von Aktuatoren als Verstelleinrichtung 76 dienen, beispielsweise ein Schrittmotor.

Zwischen der Hautoberfläche 56 und dem Objektiv 74 ist ferner ein Objektivpolarisatonsfilter 77 angeordnet, der dazu dient, unerwünschte Bestandteile des vom Untersuchungsbereich 58 reflektierten Lichts zu blockieren. Insbesondere ist die Polarisationsrichtung des Objektivpolarisationsfilters 77 senkrecht zur Polarisationsrichtung der Polarisationsfilter 52 der Beleuchtungsvorrichtungen 48.

Dadurch, dass das Objektiv 74 durch die Verstelleinrichtung 76 verfahren werden kann, kann das Objektiv 74 verschiedene Stellpositionen entlang der optischen Achse einnehmen. Somit wird, bei gleichbleibendem Objektiv 74, je nach aktueller Stellposition des Objektivs 74 eine andere Untersuchungsebene im Untersuchungsbereich 58 vom Objektiv 74 scharf auf das Sensorelement 72 abgebildet. Anders ausgedrückt definiert die Stellposition die aktuelle Untersuchungsebene der Kameraeinheit 18.

Die Verstelleinrichtung 76 ist signalübertragend mit einer Steuerungseinheit 78 verbunden, die dazu eingerichtet ist, die Verstelleinrichtung 76 zu steuern und somit die Stellposition des Objektivs 74 festzulegen.

In der Steuerungseinheit 78 sind Informationen zu zwei oder mehr vordefinierten Untersuchungsebenen im Untersuchungsbereich hinterlegt, beispielsweise den in Figur 4 dargestellten vordefinierten Untersuchungsebenen 60 und 62, jeweils mit zugehörigen Stellpositionen des Objektivs 74.

Ferner ist die Steuerungseinheit 78 signalübertragend mit der Benutzerschnittstelle 28 des Untersuchungsgeräts 10 verbunden. Somit ist die Stellposition des Objektivs 74 von der Bedienperson des Untersuchungsgeräts 10 über die Benutzerschnittstelle 28, und somit die Steuerungseinheit 78 und die Verstelleinrichtung 76, einstellbar.

Die Steuerungseinheit 78 ist weiter mit dem Sensorelement 72 signalübertragend verbunden, sodass das Sensorelement 72 von der Kameraeinheit 18 ermittelte Bilder an die Steuerungseinheit 78 übermitteln kann. Die von der Kameraeinheit 18 erhaltenen Bilder können anschließend in der Steuerungseinheit 78 gespeichert werden und über die Kommunikationsschnittstelle 42 an ein externes Gerät übertragen werden.

Im Folgenden wird die Funktionsweise des Untersuchungsgeräts 10 im Zusammenhang mit einem erfindungsgemäßen Verfahren zur Erfassung wenigstens eines Bildes des Untersuchungsbereichs 58 näher beschrieben.

In Figur 4 ist ein erstes Anwendungsszenario des Untersuchungsgeräts 10 schematisch dargestellt.

Das Untersuchungsgerät 10 ist in einem Hautbereich 80 auf der Hautoberfläche 56 eines zu untersuchenden Patienten aufgesetzt, wobei die Kontaktscheibe 54 die Hautoberfläche 56 berührt. Es ist auch möglich, dass zwischen der Hautoberfläche 56 und der Kontaktscheibe 54 eine Kontaktflüssigkeit, insbesondere ein Dermatoskopieöl, aufgebracht ist, die beispielsweise zum Angleichen der Brechungsindizes der Kameraeinheit 18 und der Hautoberfläche 56 des Patienten dient.

Der durch die gewählte Kontaktposition auf der Hautoberfläche 56 definierte Untersuchungsbereich 58 weist eine für die jeweilige Stelle am Patienten charakteristische Abfolge von Untersuchungsebenen auf, die entlang der Tiefenrichtung T übereinander angeordnet sind.

In der Steuerungseinheit 78 sind daher Informationen zu den zwei vordefinierten Untersuchungsebenen 60 und 62 hinterlegt, wobei die vordefinierte Untersuchungsebene 60 der Epidermis 64 und die vordefinierte Untersuchungsebene 62 der Dermis 66 zugeordnet ist.

Die Untersuchungsebenen sind zum Beispiel anhand ihres Abstands von der Hautoberfläche 56 in Tiefenrichtung T vordefiniert. Zum Beispiel liegt die Untersuchungsebene "Epidermis" auf dem gesunden Handrücken einer weiblichen Person bei einem Abstand von 195 µm zur Hautoberfläche.

Die Ausdehnung von Epidermis 64 und Dermis 66 am Patienten ist jedoch abhängig vom jeweils zu untersuchenden Körperteil des Patienten, sodass die Lage der vordefinierten Untersuchungsebenen 60 und 62 entlang der Tiefenrichtung T auch in Abhängigkeit des zu untersuchenden Körperteils in der Steuerungseinheit 78 hinterlegt ist, also mit weiteren Informationen zur vordefinierten Untersuchungsebene 60 bzw. 62 verknüpft ist.

Die Informationen zu den vordefinierten Untersuchungsebenen 60 und 62 umfassen daher insbesondere Angaben zum Hauttyp, zum Körperteil, zur Art des Hautbereichs 80, zur Art der vermuteten Läsion und/oder zur Position des zu untersuchenden Hautbereichs 80.

Die entsprechenden Informationen zur Untersuchungsebene "Epidermis" des vorgenannten Beispiels enthalten daher die Angaben "weiblich" zum Hauttyp, "Hand" zum Körperteil, "gesund" zur Art des Hautbereichs 80 und "Handrücken" zur Position des zu untersuchenden Hautbereichs 80. Zudem ist mit der Untersuchungsebene "Epidermis" der Abstand zur Hautoberfläche von 195µm verknüpft, z.B. als Teil der Informationen.

Beispielsweise handelt es sich bei dem in Fig. 4 gezeigten Hautbereich 80 um einen erkrankten Bereich am Rücken eines männlichen Patienten. Derartige Hautbereiche weisen eine vergleichsweise dünne Epidermis 64 und eine vergleichsweise dicke Dermis 66 auf.

Bei dem in Fig. 5 gezeigten Hautbereich 80, der einem zweiten Anwendungsszenario entspricht, handelt es sich hingegen beispielsweise um einen gesunden Bereich an der Oberseite einer Hand eines männlichen Patienten und damit um einen Hautbereich, der eine vergleichsweise dicke Epidermis 64 und eine vergleichsweise dünne Dermis 66 im Vergleich zur Situation in Fig. 4 aufweist.

Dementsprechend sind in der Steuerungseinheit 78 für diese unterschiedlichen Anwendungsszenarien unterschiedliche vordefinierte Untersuchungsebenen 60 bzw. 62 hinterlegt, die jeweils sicherstellen, dass die Untersuchungsebenen 60 bzw. 62 im gewünschten Bereich des Untersuchungsbereichs 58 liegen und eine korrekte Darstellung liefern.

Zu jeder der vordefinierten Untersuchungsebenen ist in der Steuerungseinheit 78 zudem eine Stellposition des Objektivs 74 hinterlegt, sodass die Steuerungseinheit 78 bei Auswahl einer der vordefinierten Untersuchungsebenen 60 bzw. 62 das Objektiv 74 entlang der optischen Achse mittels der Verstelleinrichtung 76 verfahren kann, derart, dass das Objektiv 74 in der resultierenden Stellposition die jeweilige vordefinierte Untersuchungsebene 60 bzw. 62 scharf auf das Sensorelement 72 abbildet.

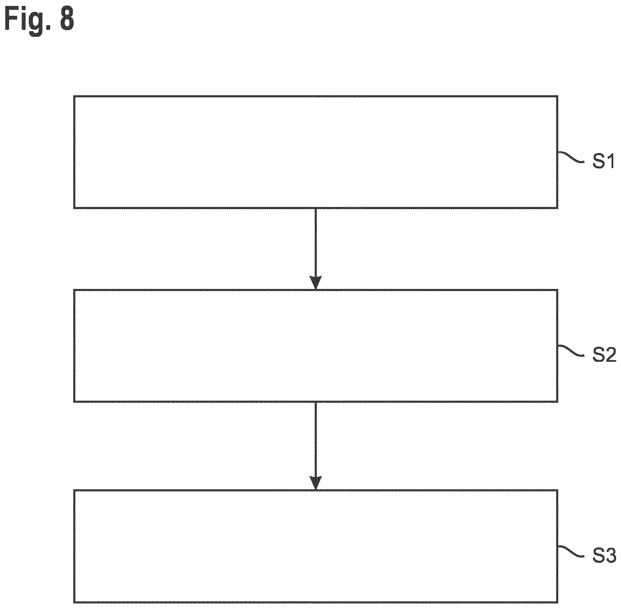

Im erfindungsgemäßen Verfahren zur Erfassung eines Bildes des Untersuchungsbereichs 58 wird zunächst eine Untersuchungsebene ausgewählt (Schritt S1 in Fig. 8), wobei insbesondere eine der vordefinierten Untersuchungsebenen 60 und 62 ausgewählt wird.

Dies kann durch die Bedienperson des Untersuchungsgeräts 10 über die Benutzerschnittstelle 28 geschehen, wobei insbesondere auf der Anzeige 26 die zur Verfügung stehenden Optionen angezeigt werden. Beispielsweise werden als Untersuchungsebenen "Dermis" und "Epidermis" angeboten.

Die angezeigten Optionen können auf bestimmte Anwendungsszenarien eingeschränkt sein, um die Bedienung des Untersuchungsgeräts 10 zu vereinfachen.

Es ist auch möglich, dass die Untersuchungsebene automatisiert von der Steuerungseinheit 78 gewählt wird, indem das Untersuchungsgerät 10 mit der Kontaktscheibe 54 auf den Hautbereich 80 aufgesetzt und mittels der Kameraeinheit 18 wenigstens ein Bild aufgenommen wird, anhand dem die Steuerungseinheit 78 die notwendigen Informationen zum Untersuchungsbereich 58 ermittelt, also beispielsweise um welchen Hauttyp, welchen Körperteil, welche Art des Hautbereichs 80 und/oder um welche Position des Hautbereichs 80 es sich handelt. Auch kann die Steuereinheit 78 eine Vermutung zu einer Läsion, die eine Information zum Untersuchungsbereich 58 darstellen kann, anhand des aufgenommenen Bildes vornehmen.

Bei der Aufnahme dieses Bildes ist die Kameraeinheit 18 auf die äußere Oberfläche der Kontaktscheibe 54 bzw. der Hautoberfläche 56 fokussiert, also scharfgestellt.

Zu diesem Zweck können in der Steuerungseinheit 78 Referenzbilder hinterlegt sein bzw. werden, die mit dem wenigstens einen Bild verglichen werden. Sobald die Informationen zu den Untersuchungsebenen auf diese Weise automatisiert ermittelt wurden, kann der Bedienperson über die Benutzerschnittstelle 28 eine Auswahl der vordefinierten Untersuchungsebenen 60 und 62 präsentiert werden, aus denen sie wählt. Alternativ kann die vordefinierte Untersuchungsebene 60 bzw. 62 ebenfalls automatisiert von der Steuerungseinheit 78 ausgewählt werden.

In einer weiteren Variante kann mittels der zweiten Kameraeinheit 20 ein Übersichtsbild des Hautbereichs 80, in dem der Untersuchungsbereich 58 gebildet werden soll, aufgenommen werden, das von der Steuerungseinheit 78 zum Ermitteln der Informationen der vordefinierten Untersuchungsebene herangezogen wird.

Sobald eine vordefinierte Untersuchungsebene ausgewählt wurde, beispielsweise die vordefinierte Untersuchungsebene 60, sendet die Steuerungseinheit 78 ein Einstellsignal an die Verstelleinrichtung 76, die wiederum das Objektiv 74 der Kameraeinheit 18 auf eine der ausgewählten Untersuchungsebene zugeordnete Stellposition entlang der optischen Achse verfährt (Schritt S2 in Fig. 8).

Die zugeordnete Stellposition ist diejenige Stellposition des Objektivs 74, in der die ausgewählte Untersuchungsebene scharf auf dem Sensorelement 72 abgebildet wird.

Somit kann anschließend wenigstens ein Bild der ausgewählten Untersuchungsebene, beispielsweise der vordefinierten Untersuchungsebene 60, mittels des Sensorelements 72 der Kameraeinheit 18 aufgenommen werden (Schritt S3 in Fig. 8).

Durch die Zuordnung der Stellpositionen zu den vordefinierten Untersuchungsebenen und den weiteren damit verknüpften Informationen wie dem Hautbereich ist auf diese Weise eine einfache und zuverlässige Möglichkeit zum Aufnehmen von Bildern des Patienten möglich.

Das aufgenommene Bild kann der Bedienperson auf der Anzeige 26 dargestellt werden, insbesondere als Livedarstellung.

Es ist auch möglich, dass die Bedienperson über die Benutzerschnittstelle 28, insbesondere eines der Bedienelemente 30, die Stellposition des Objektivs 74 und damit die Untersuchungsebene 82 selbstständig einstellt oder anpasst.

Beispielsweise kann die Bedienperson die Untersuchungsebene um einen vorgegebenen Wert oder ein Vielfaches des vorgegebenen Wertes durch Eingabe an der Benutzerschnittstelle, z.B. durch Betätigen einer Taste verschieben. Die Steuereinheit 72 verändert dann die Stellposition des Objektivs 74 um den vorgegebenen Wert oder ein Vielfaches des vorgegebenen Wertes. Auf diese Weise können ein oder mehrerer Bilder weiterer Untersuchungsebenen 82 erhalten werden, die in einem festgelegten Abstand di von einer vordefinierten Untersuchungsebenen entfernt sind. Die Bedienperson ist somit nicht auf die vordefinierten Untersuchungsebenen beschränkt.

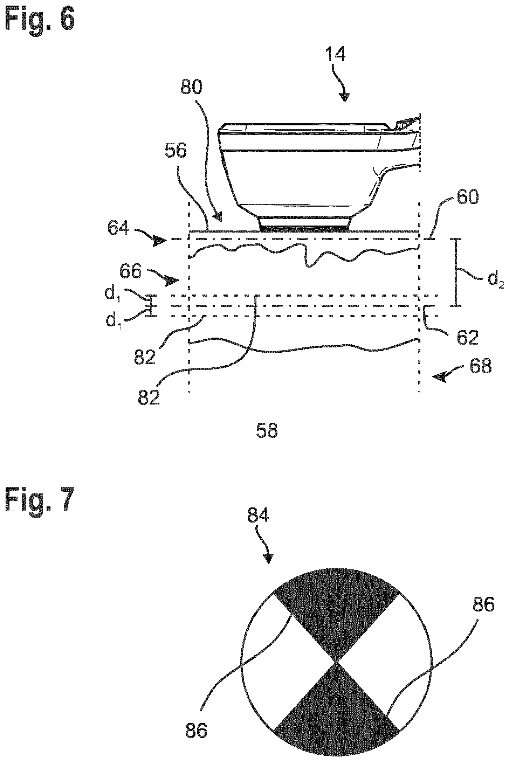

In Figur 6 sind zwei derartige weiterer Untersuchungsebenen 82 mit Strichlinien angedeutet, die um die vordefinierte Untersuchungsebene 62 herum angeordnet sind. Im Übrigen entspricht das Anwendungsszenario aus Figur 6 demjenigen aus Figur 4.

Der festgelegte Abstand di ist insbesondere kleiner als ein Abstand d₂, der durch die vordefinierten Untersuchungsebenen 60 und 62 definiert ist.

Es ist auch möglich, dass die Bedienperson über die Benutzerschnittstelle 28 eine manuelle stufenlose Veränderung der Stellposition des Objektivs 74 vornehmen kann.

Das Untersuchungsgerät 10 verfügt ferner über einen Kalibrierungsmodus, in dem das Untersuchungsgerät 10 auf eine Referenzfläche 84 aufgesetzt ist. In Fig. 7 ist eine beispielhafte Referenzfläche 84 gezeigt. Die Referenzfläche 84 ist zum Beispiel auf eine Kalibrierungskarte gedruckt, die mit dem Untersuchungsgerät 10 ausgeliefert wird.

Die Referenzfläche 84 verfügt über mindestens eine, bevorzugt mehrere, Targets 86 bzw. Kontrastelemente.

Zur Kalibrierung wird das Untersuchungsgerät 10 mit der Kontaktscheibe 54 auf die Referenzfläche 84 aufgelegt. Die Außenseite der Kontaktscheibe entspricht in diesem Fall der Kalibrierungsebene.

Das Objektiv 74 wird dann so lange von der Steuerungseinheit 78 bzw. der Verstelleinrichtung 76 in unterschiedliche Positionen verfahren, bis das mindestens eine Kontrastelement 86 mit einem maximalen Kontrast im aufgenommenen Bild der Referenzfläche 84 dargestellt wird.

Die auf diese Weise ermittelte Stellposition für die Kalibrierung, d.h. die Stellposition der Verstelleinrichtung 76, die der Kalibrierungsebene zugeordnet ist, wird als die Stellposition angesehen, die der Hautoberfläche zugeordnet ist.

Die zwei oder mehr Stellpositionen, die den vordefinierten Untersuchungsebenen 60 und 62 zugeordnet sind, können daraufhin durch die Steuerungseinheit 78 bestimmt werden.

Beispielsweise wird hierzu der zu den vordefinierten Untersuchungsebenen hinterlegte Abstand zur Hautoberfläche herangezogen und anhand dieses Abstandes die zu den vordefinierten Untersuchungsebenen zugehörigen Stellpositionen relativ zu der zur Hautoberfläche zugehörigen Stellposition (Stellposition für die Kalibrierung) berechnet.

Es ist auch möglich, dass die auf diese Weise ermittelte zur Hautoberfläche zugehörige Stellposition bereits eine Stellposition ist, die einer der vordefinierten Untersuchungsebenen 60 und 62 zugeordnet ist.

Über das von der Referenzfläche 84 aufgenommene Bild kann ferner ein Weißabgleich vorgenommen werden. Mit anderen Worten kann über einen Vergleich des von der Referenzfläche 84 aufgenommenen Bildes mit einem Vergleichswert bzw. einem Referenzbild eine Abweichung der Farbdarstellung von einem Soll-Wert ermittelt und in der Steuerungseinheit 78 hinterlegt werden. Im Einsatz des Untersuchungsgeräts 10 erhaltene Bilder können anschließend anhand der bestimmten Abweichung korrigiert werden, um möglichst farbechte Darstellungen des Untersuchungsbereichs 58 zu erhalten.

Insgesamt zeichnet sich das erfindungsgemäße Untersuchungsgerät 10 durch eine einfache Bedienung aus und ermöglicht zugleich eine zuverlässige Aufnahme von Bildern vordefinierter Untersuchungsebenen eines Patienten.

## Patentansprüche

1. Untersuchungsgerät (10) für optische medizinische Untersuchungen, insbesondere Dermatoskop, mit einer Kameraeinheit (18) zum Aufnehmen wenigstens eines Bildes eines Untersuchungsbereichs (58) eines Patienten, einer Anzeigevorrichtung (22) und einer Steuerungseinheit (78),
wobei die Kameraeinheit (18) ein Sensorelement (72), ein Objektiv (74) und eine Verstelleinrichtung (76) für das Objektiv (74) aufweist, wobei das Objektiv (74) in einem Beobachtungsstrahlengang (70) der Kameraeinheit (18) vor dem Sensorelement (72) angeordnet ist,
wobei die Verstelleinrichtung (76) dazu eingerichtet ist, das Objektiv (74) entlang der optischen Achse des Objektivs (74) in verschiedene Stellpositionen zu verfahren, um verschiedene Untersuchungsebenen im Untersuchungsbereich (58) zu fokussieren,
wobei die Steuerungseinheit (78) zur Steuerung der Verstelleinrichtung (76) ausgebildet und mit dieser verbunden ist, und in der Steuerungseinheit (78) Informationen zu zwei oder mehr vordefinierten Untersuchungsebenen (60, 62) im Untersuchungsbereich (58) mit zugehörigen Stellpositionen des Objektivs (74) hinterlegt sind, und
wobei die Anzeigevorrichtung (22) dazu eingerichtet ist, eine Darstellung des von der Kameraeinheit (18) aufgenommenen wenigstens einen Bildes anzuzeigen.

2. Untersuchungsgerät (10) nach Anspruch 1, wobei der Untersuchungsbereich (58) ein Hautbereich des Patienten ist.

3. Untersuchungsgerät (10) nach Anspruch 2, wobei insbesondere die Informationen zu den Untersuchungsebenen Angaben zum Hauttyp, zum Körperteil, zur Art des Hautbereichs (80), zur Art der vermuteten Läsion und/oder zur Position eines zu untersuchenden Hautbereichs (80) umfassen.

4. Untersuchungsgerät (10) nach Anspruch 3, wobei das Untersuchungsgerät (10) eine Benutzerschnittstelle (28) umfasst, mit der der Hauttyp, das zu untersuchende Körperteil, die Art des Hautbereichs (80), die Art der vermuteten Läsion und/oder die Position des zu untersuchenden Hautbereichs (80) auswählbar ist, und die vordefinierten Untersuchungsebenen (60, 62) in Abhängigkeit des ausgewählten wenigstens einen Hauttyps, des zu untersuchenden Körperteils, der Art des Hautbereichs (80), der Art der vermuteten Läsion und/oder der Position des zu untersuchenden Hautbereichs (80) ausgewählt sind.

5. Untersuchungsgerät (10) nach Anspruch 3 oder 4, wobei das Untersuchungsgerät (10) dazu eingerichtet ist den Hauttyp, das zu untersuchende Körperteil, die Art des Hautbereichs (80), die Art der vermuteten Läsion und/oder die Position des zu untersuchenden Hautbereichs (80) zu erkennen und/oder auszuwählen, insbesondere anhand eines aufgenommenen Bildes der Kameraeinheit (18) oder eines aufgenommenen Bildes einer zweiten Kameraeinheit (20) des Untersuchungsgerätes (10).

6. Untersuchungsgerät (10) nach einem der Ansprüche 3 bis 5, wobei der wenigstens eine Hauttyp durch das Körperteil, die Art des Hautbereichs (80) und/oder die Position des Hautbereichs (80) am Patienten vorgegeben ist.

7. Untersuchungsgerät (10) nach einem der vorherigen Ansprüche, wobei die Verstelleinrichtung (76) ein Aktuator ist, insbesondere ein Schrittmotor oder ein Voice-Coil-Motor.

8. Untersuchungsgerät (10) nach einem der vorhergehenden Ansprüche, wobei das Untersuchungsgerät (10) eine Benutzerschnittstelle (28) umfasst, mit der eine der vordefinierten Untersuchungsebenen (60, 62) auswählbar ist, und die Steuerungseinheit (78) dazu ausgebildet ist, das Objektiv (74) in die zur gewählten Untersuchungsebene zugehörige Stellposition zu verfahren, insbesondere wobei lediglich solche vordefinierten Untersuchungsebenen (60, 62) zur Auswahl über die Benutzerschnittstelle (28) angeboten werden und/oder auswählbar sind, die dem zuvor ausgewähltem Hauttyp, Körperteil, Art der vermuteten Läsion, Art und/oder Position eines zu untersuchenden Hautbereichs (80) zugeordnet sind.

9. Untersuchungsgerät (10) nach Anspruch 8, wobei die Benutzerschnittstelle (28) wenigstens eine Taste und/oder ein berührungsempfindliches Display umfasst.

10. Untersuchungsgerät (10) nach einem der vorhergehenden Ansprüche, wobei die Anzeigevorrichtung (22) dazu eingerichtet ist, die Darstellung des von der Kameraeinheit (18) aufgenommenen wenigstens einen Bildes als Livedarstellung anzuzeigen.

11. Untersuchungsgerät (10) nach einem der vorhergehenden Ansprüche, wobei das Untersuchungsgerät (10) eine Benutzerschnittstelle (28) umfasst, mittels der die Stellposition der Verstelleinrichtung (76) derart einstellbar ist, dass die Stellposition der Verstelleinrichtung (76) um einen vorgegebenen Wert oder ein Vielfaches des vorgegebenen Werts von der aktuellen Stellposition verändert wird.

12. Untersuchungsgerät (10) nach einem der vorhergehenden Ansprüche, wobei über die Benutzerschnittstelle (28) die Stellposition der Verstelleinrichtung (76) manuell stufenlos justierbar ist.

13. Untersuchungsgerät (10) nach einem der vorhergehenden Ansprüche, wobei die Steuerungseinheit (78) dazu eingerichtet ist, in einem Kalibrierungsmodus, in dem das Untersuchungsgerät (10) auf eine Referenzfläche (84) aufgesetzt ist, die zwei oder mehr Stellpositionen anhand einer Autofokussierung zu ermitteln, und/oder einen Weißabgleich anhand wenigstens eines mittels der Kameraeinheit (18) aufgenommenen Bildes der Referenzfläche (84) durchzuführen.

14. Verfahren zur Erfassung eines Bildes eines Untersuchungsbereichs (58) mittels eines Untersuchungsgeräts (10), insbesondere eines Untersuchungsgeräts (10) nach einem der vorhergehenden Ansprüche, umfassend:
- Auswählen einer Untersuchungsebene,
- Verstellen eines Objektivs (74) einer Kameraeinheit (18) des Untersuchungsgeräts (10) auf eine der ausgewählten Untersuchungsebene zugeordneten Stellposition,
- Aufnehmen wenigstens eines Bildes mittels eines Sensorelementes (72) der Kameraeinheit (18).

15. Verfahren nach Anspruch 14, wobei das Auswählen der Untersuchungsebene durch den Benutzer oder durch das Untersuchungsgerät (10) erfolgt.
